# EUROPEAN PATENT SPECIFICATION

(11) **EP 1 180 351 B1**
(45) Date of publication and mention of the grant of the patent: **23.01.2008**
(21) Application number: 01306198.1
(22) Date of filing: 19.07.2001
(51) Int. Cl.: A61F 2/08

(54) **Apparatus for reconstructing a ligament**
Gerät zum Wiederherstellen einer Sehne
Appareil pour reconstruire un ligament

(30) Priority: 19.07.2000 US 619105
(43) Date of publication of application: 20.02.2002
(73) Proprietor: DePuy Mitek, Inc., Raynham, MA 02767-0350 (US)
(72) Inventor: Goble, E. Marlowe, Alta, Wyoming 83452 (US); Chervitz, Alan, Palm Harbor, Florida 34685 (US); Justin, Daniel F., Logan, Utah 84321 (US); Fallin, T. Wade, Hyde Park, Utah 84318 (US)
(74) Representative: Thomson, Paul Anthony

(56) References cited:
- EP-A- 0 376 641
- EP-A- 0 496 140
- EP-A- 0 688 185
- EP-A- 0 860 146
- GB-A- 2 337 463
- US-A- 5 918 604

## Description

### Field Of The Invention

This invention relates to a medical device for reconstructing a ligament.

### Background Of The Invention

A ligament is a piece of fibrous tissue which connects one bone to another.

Ligaments are frequently damaged (e.g., detached or torn or ruptured, etc.) as the result of injury and/or accident. A damaged ligament can impede proper motion of a joint and cause significant pain.

Various procedures have been developed to repair or replace a damaged ligament. The specific procedures used depend on the particular ligament which is to be restored and on the extent of the damage.

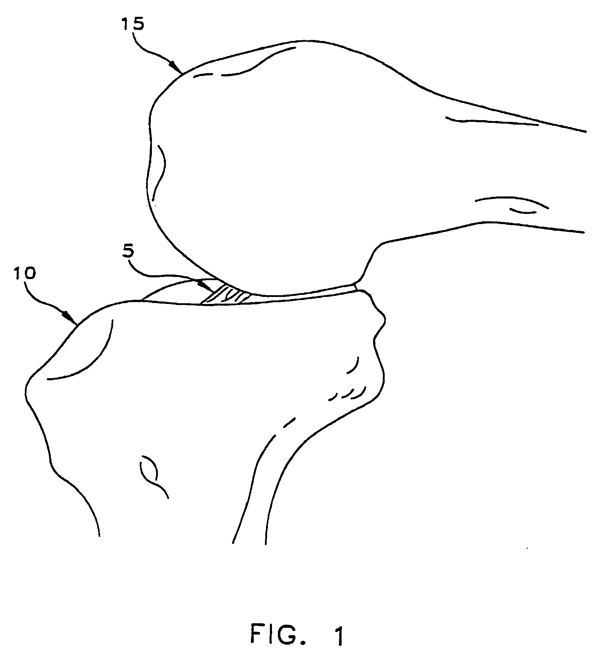

One ligament which is frequently damaged as the result of injury and/or accident is the anterior cruciate ligament (ACL). Looking now at Fig. 1, the ACL 5 extends between the top of the tibia 10 and the bottom of the femur 15. A damaged ACL can cause instability of the knee joint and cause substantial pain and arthritis.

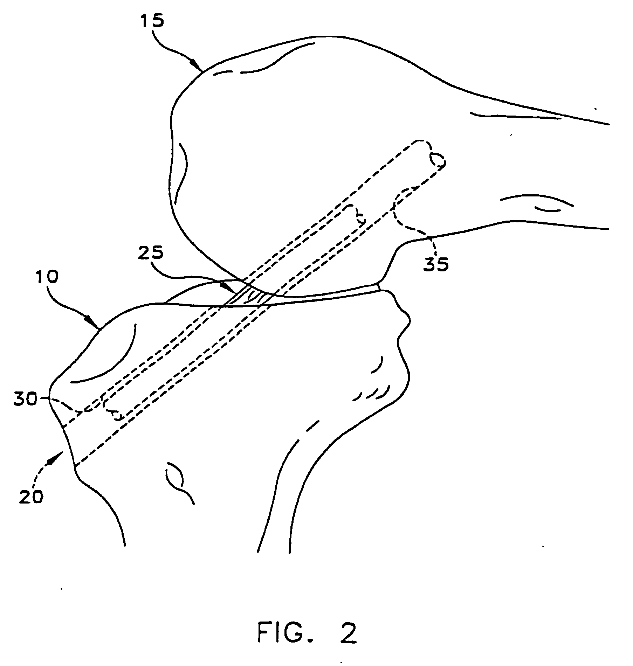

Numerous procedures have been developed to restore the ACL through a graft ligament replacement. In general, and looking now at Fig. 2, these ACL replacement procedures involve drilling a bone tunnel 20 through tibia 10 and up into femur 15. Then a graft ligament 25, consisting of a harvested or artificial ligament or tendon(s), is passed through the tibial portion 30 of tunnel 20 (sometimes referred to as "the tibial tunnel"), across the interior of the joint, and up into the femoral portion 35 of tunnel 20 (sometimes referred to as "the femoral tunnel"). Then a distal portion of graft ligament 25 is secured in femoral tunnel 35, and a proximal portion of graft ligament 25 is secured in tibial tunnel 30.

### Objects Of The Invention

One object of the present invention is to provide improved apparatus for positioning the graft ligament in the bone tunnel and/or for securing the graft ligament within the bone tunnel.

### Summary Of The Invention

A crosspin according to the preamble of claim 1 is known from the document EP-A-0376641. are addressed by

The invention comprises a crosspin for supporting a graft ligament in a first bone tunnel formed in a bone, by positioning the cross pin in a second bone tunnel extending transverse to, and intersecting, the first bone tunnel, the crosspin comprising:
a shaft having a distal portion, an intermediate portion, and a proximal portion;
the distal portion comprising attachment means for attaching a flexible member to said shaft;
the intermediate portion comprising screw threads; and
the proximal portion including driver engagement means for engagement by a driver adapted to turn said shaft;
whereby the crosspin may be drawn through the second transverse bone tunnel by the flexible member attached to the distal portion by said attachment means, and
the crosspin being turnable to have screw threads thereof turned into bone by the driver engaged with the engagement means;
**characterized by**
a male/female screw mount for releasably connecting the distal portion to the intermediate portion, such that the distal portion is separable from the intermediate portion.

### Brief Description Of The Drawings

The above and other objects and features of the present invention will be more fully disclosed or rendered obvious by the following detailed description of the preferred embodiments of the invention, which is to be considered together with the accompanying drawings wherein like numbers refer to like parts, and further wherein:
Fig. 1 is a schematic side view of a knee joint, showing an ACL extending between the top of the tibia and the bottom of the femur;
Fig. 2 is a schematic side view of the same knee joint, except showing portions of an ACL reconstruction;
Figs. 3-7 are schematic front views of a knee joint, illustrating a crosspinning procedure utilizing a first type of crosspin;
Fig. 8 is a schematic front view of a knee joint, illustrating a crosspinning procedure utilizing a second type of crosspin;
Fig. 9 is a schematic front view of a knee joint, illustrating a crosspinning procedure
Figs. 10-12 are schematic views illustrating a crosspinning procedure utilizing a graft ligament comprising a bone block;
Figs. 13-15 are schematic views illustrating another crosspinning procedure utilizing a graft ligament comprising a bone block;
Figs. 16-18 are schematic views illustrating still another crosspinning procedure utilizing a graft ligament comprising a bone block;
Figs. 19-21 are schematic views illustrating a crosspinning procedure utilizing a plate and a graft ligament comprising a bone block; and
Fig. 22 is a schematic view illustrating another crosspinning procedure utilizing a plate and a graft ligament comprising a bone block.

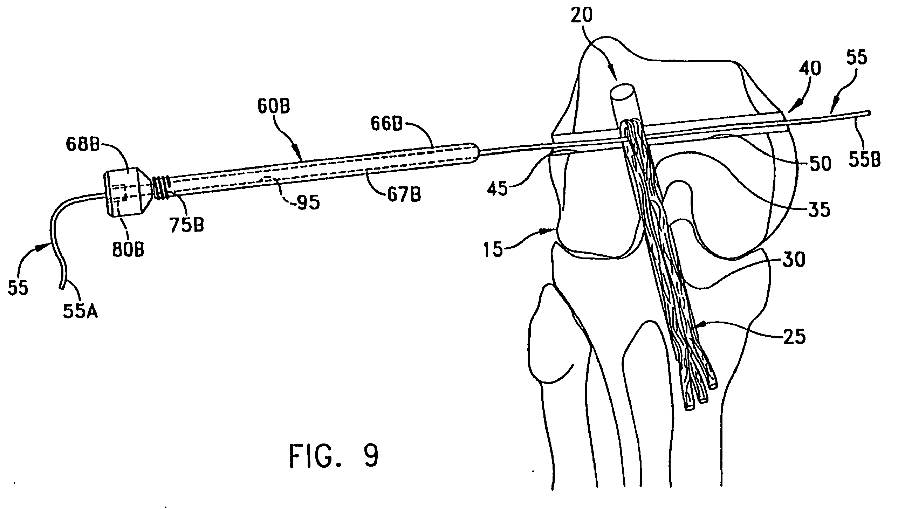

Figures 9 to 22 do not disclose embodiments of the invention.

### Detailed Description Of The Invention.

### Non-Cannulated Crosspin

In one form of the invention, there is provided a novel non-cannulated crosspin for supporting a graft ligament in a bone tunnel.

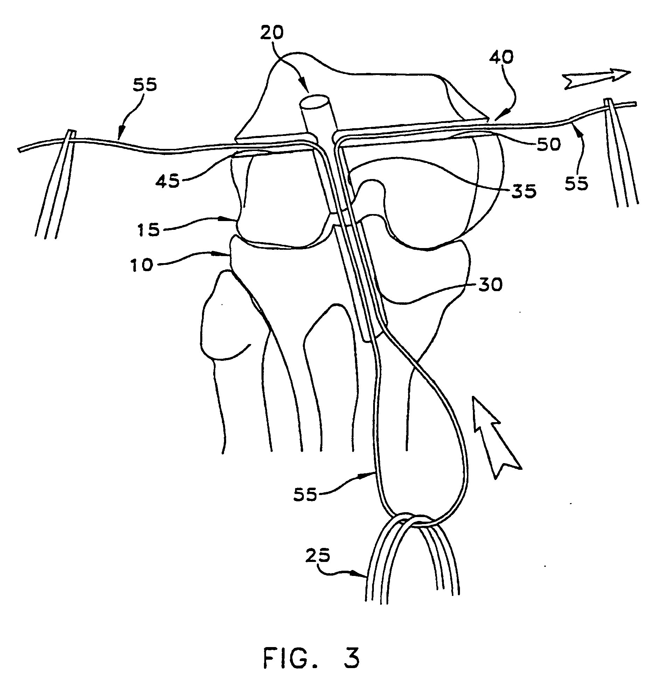

More particularly, and looking now at Fig. 3, during one type of ACL reconstruction, the bone tunnel 20 is formed by drilling through tibia 10 and up into femur 15, whereby to form tibial tunnel 30 and femoral tunnel 35. Then a transverse bone tunnel 40 is formed in femur 15 so that transverse bone tunnel 40 intersects femoral tunnel 35. Bone tunnel 20 bifurcates transverse bone tunnel 40 into two tunnel portions, a first transverse bone tunnel portion 45 and a second transverse bone tunnel portion 50.

After transverse bone tunnel 40 has been formed, a flexible member 55 is used to draw graft ligament 25 up into place.

There are a number of ways that this may be accomplished and, for the purposes of the present invention, all are satisfactory. However, for purposes of example but not limitation, a particular method for drawing graft ligament 25 into place using flexible member 55 will now be reviewed.

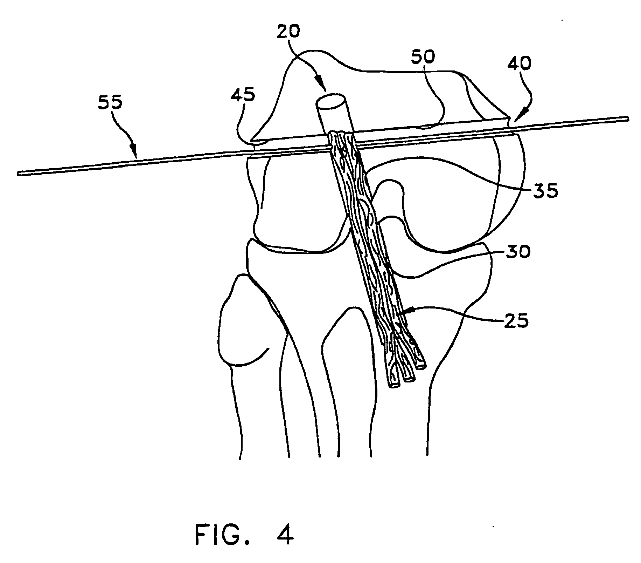

First, flexible member 55 is threaded through transverse bone tunnel 40. Then a crochet-hook device (not shown) is passed up tibial tunnel 30, across the interior of the knee joint, and up femoral tunnel 35. The crochet-hook device is used to hook flexible member 55 at the intersection of bone tunnel 20 and transverse bone tunnel 40. Then the crochet-hook device is used to pull flexible member 55 down femoral tunnel 35, across the interior of the knee joint, down tibial tunnel 30, and out the front side of tibia 10. Next, graft ligament 25 is looped over flexible member 55 (Fig. 3). If desired, graft ligament 25 can be secured around flexible member 55 by a suture, a clip or a tie device (not shown) so as to prevent graft ligament 25 from slipping off flexible member 55. Flexible member 55 is then used to pull the looped graft ligament 25 up tibial tunnel 30, across the interior of the knee joint, and then up into femoral tunnel 35 (Fig. 4).

Once graft ligament 25 and flexible member 55 have assumed the position shown in Fig. 4, the graft ligament may be retained in that position through the use of a novel non-cannulated crosspin.

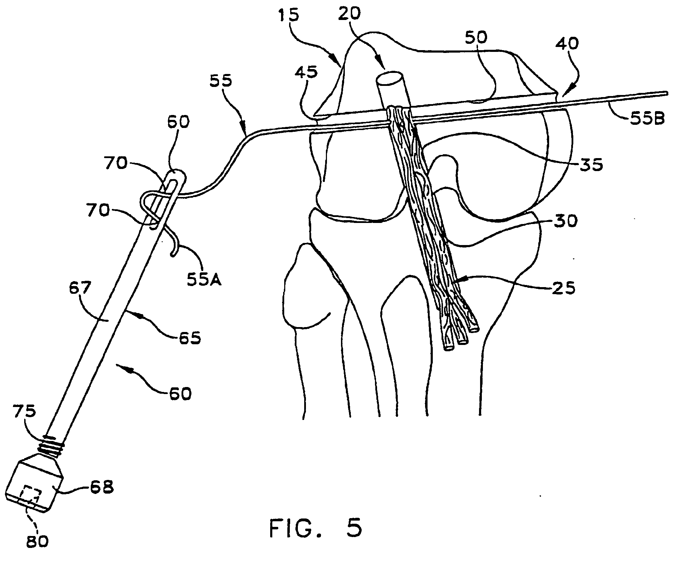

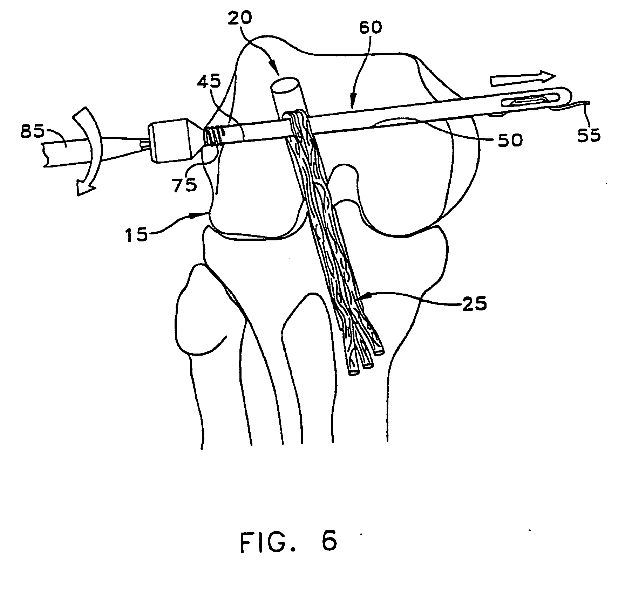

More particularly, and looking now at Figs. 5-7, graft ligament 25 may be supported in femoral tunnel 35 with a novel crosspin 60. Crosspin 60 generally comprises a solid shaft 65 having a distal portion 66, an intermediate portion 67 and a proximal portion 68. One or more openings 70 are formed in the shaft's distal portion 66, and screw threads 75 are formed in the shaft's intermediate portion 67, adjacent to proximal portion 68. Crosspin 60 also comprises a recess 80 in its proximal portion 68 for receiving the front end of a driver 85 (Fig. 6).

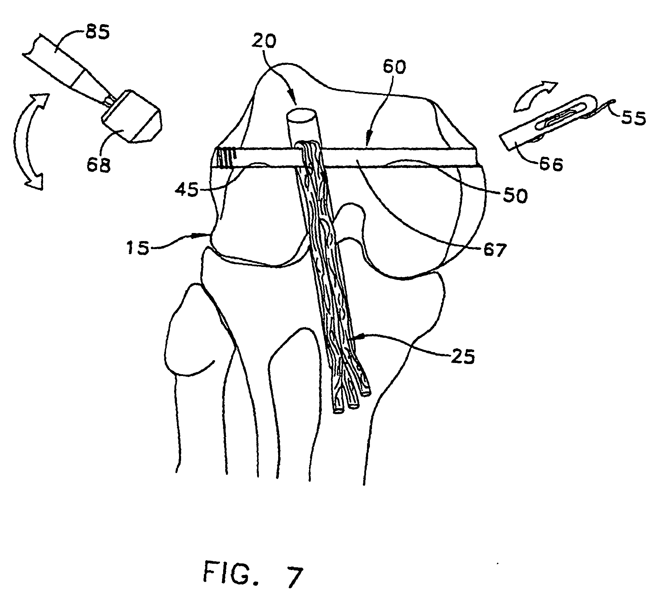

Crosspin 60 is deployed by (1) attaching one end, 55A, of flexible member 55 to crosspin 60 using openings 70 (Fig. 5); (2) drawing crosspin 60 across first transverse bone tunnel portion 45, under the looped graft ligament 25, across second transverse bone tunnel portion 50, and out the far side of transverse bone tunnel 40, until the crosspin's screw threads 75 engage femur 15; (3) turning crosspin 60 with driver 85 so that threads 75 are set into femur 15 (Fig. 6); and (4) removing the distal and proximal portions of crosspin 60 that extend beyond the outside surfaces of femur 15 (Fig. 7).

### Non-Cannulated Crosspin

### With Pre-Attached Flexible Member

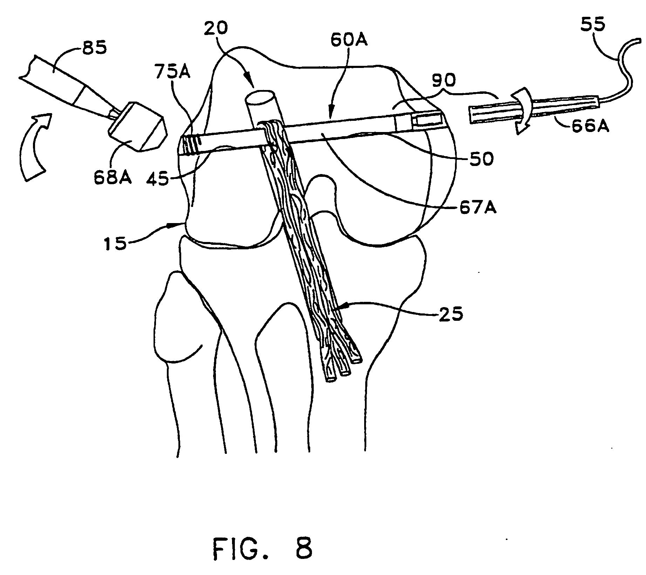

Looking next at Fig. 8, there is shown a novel non-cannulated crosspin 60A which is similar to the crosspin 60 described above, except that the distal portion 66A of crosspin 60A has flexible member 55 permanently attached thereto. Thus, with crosspin 60A, flexible member 55 does not need to be connected to the crosspin at the time of use, as is the case with the crosspin 60 discussed above. With this one exception, crosspin 60A is intended to be used in substantially the same way as the crosspin 60 discussed above.

Moreover, the distal portion 66A of crosspin 60A is joined to intermediate portion 67A of the crosspin through a male/female screw mount, such as is shown generally at 90. Such a feature facilitates removal of distal portion 66A from intermediate portion 67A after the crosspin has been set in femur 15.

All the above disclosed cross pins incorporate this screw mount.

### Cannulated Crosspin

Looking next at Fig. 9, there is shown cannulated crosspin 60B not part of the present invention. Crosspin 60B is identical to the crosspin 60 discussed above, except that (1) crosspin 60B lacks the openings 70 formed in the distal portion 66 of the crosspin 60 discussed above, and (2) crosspin 60B is cannulated along its length with a longitudinal bore 95.

In use, flexible member 55 and graft ligament 25 are first positioned in the manner shown in Fig. 4, and then crosspin 60B is slipped over the free end 55A of flexible member 55, with flexible member 55 being received in the crosspin's longitudinal bore 95. Crosspin 60B is then advanced along flexible member 55 so that the crosspin passes through first transverse bone tunnel portion 45, under the looped graft ligament 25, and through second transverse bone tunnel portion 50, until the crosspin's threads 75B engage the outer surface of femur 15. A cannulated driver (not shown, but similar to the driver 85 shown in Figs. 6-8, except that it is cannulated) is then loaded over the free end 55A of flexible member 55, advanced along flexible member 55, and then used to advance crosspin 60B so that the crosspin's screw threads 75B are set in femur 15. The cannulated driver is then withdrawn, flexible member 55 is removed, and the crosspin's protruding distal portion 66B and proximal portion 60B trimmed off so as to complete the crosspinning procedure.

### Crosspinning A Graft Ligament.

### Comprising A Bone Block

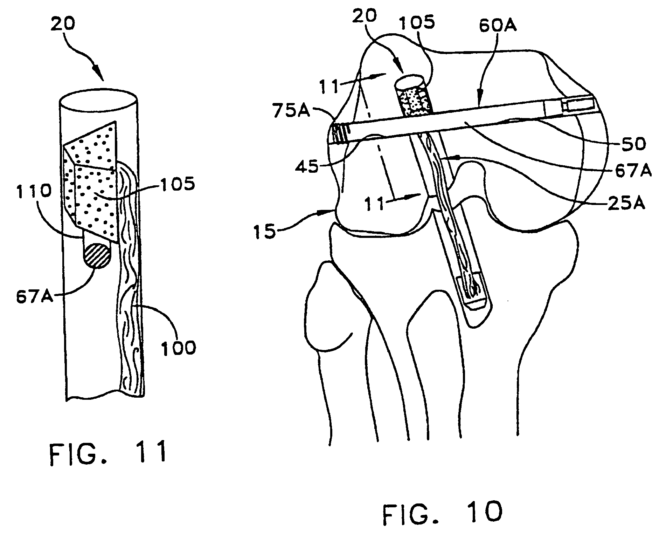

Looking now at Figs. 10-18, there is disclosed a novel method for crosspinning a graft ligament comprising a bone block. The method is not part of the invention.

More particularly, in the preceding sections, the present invention has been discussed in the context of a graft ligament 25 comprising a loop of soft tissue, e.g., a hamstring tendon. However, it is also possible to crosspin a graft ligament comprising a bone block.

More particularly, and looking now at Figs. 10 and 11, there is shown a graft ligament 25A comprising a ligament 100 and a bone block 105. By way of example but not limitation, graft ligament 25A might be a patellar graft comprising a portion of the patella tendon and a portion of the patella. Such graft ligaments are sometimes preferred since ligament 100 is naturally, and hence securely, attached to bone block 105, and since it is relatively easy to achieve good osseointegration between bone block 105 and femur 15.

Graft ligament 25A is positioned in bone tunnel 20 so that bone block 105 resides distal to crosspin 60A, i.e., so that bone block 105 resides outboard of crosspin 60A relative to the interior of the joint. As a result, when graft ligament 25A is thereafter placed under tension, crosspin 60A will prevent the bone block 105 from passing by the crosspin, whereby graft ligament 25A will be maintained in position.

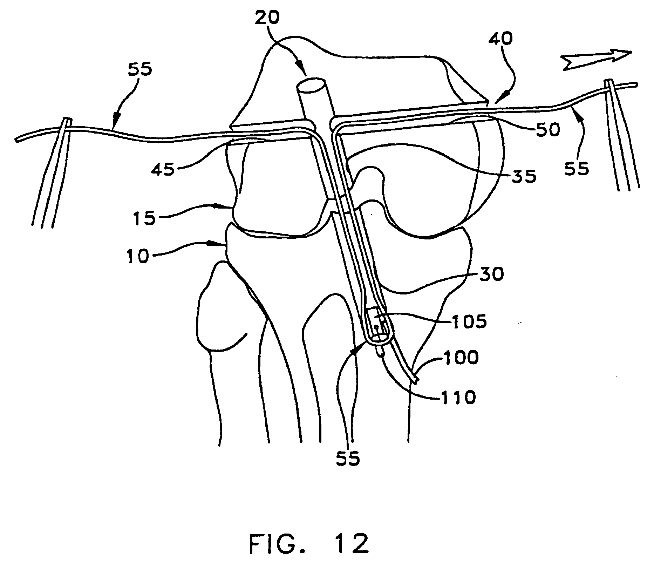

Fig. 12 illustrates how graft ligament 25A may be loaded onto flexible member 55 so that it achieves the position shown in Figs. 10 and 11. If desired, a suture 110 (Figs. 11 and 12) may be used to help keep graft ligament 25A properly positioned relative to flexible member 55 and, subsequently, crosspin 60A.

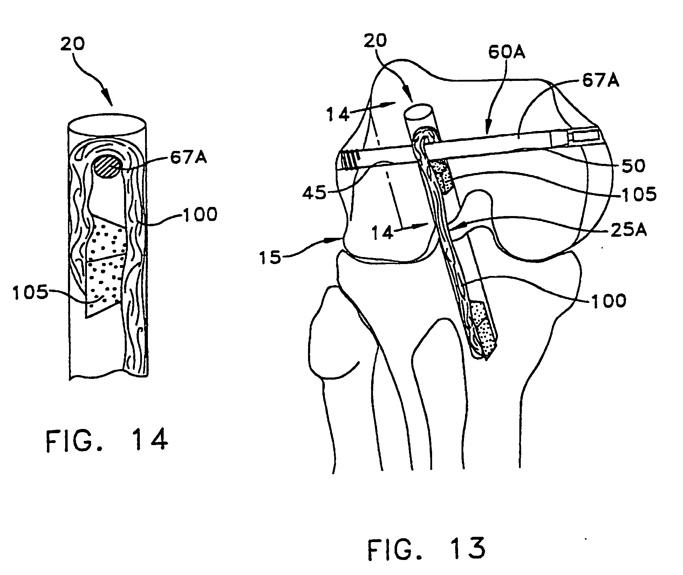

Similarly, and looking now at Figs. 13 and 14, the graft ligament 25A may also be secured in position by positioning bone block 105 proximal to crosspin 60A, with ligament 100 looping over crosspin 60A before passing proximally out of femoral tunnel 35. As a result, when graft ligament 25A is thereafter placed under tension, crosspin 60A will prevent the bone block 105 from passing by the crosspin, whereby graft ligament 25A will be maintained in position.

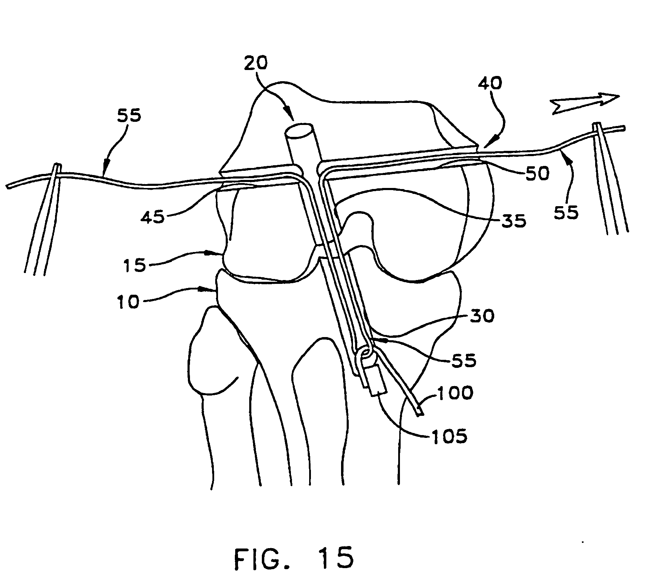

Fig. 15 illustrates how graft ligament 25A may be loaded onto flexible member 55 so that it subsequently achieves the position shown in Figs. 13 and 14 with respect to crosspin 60A.

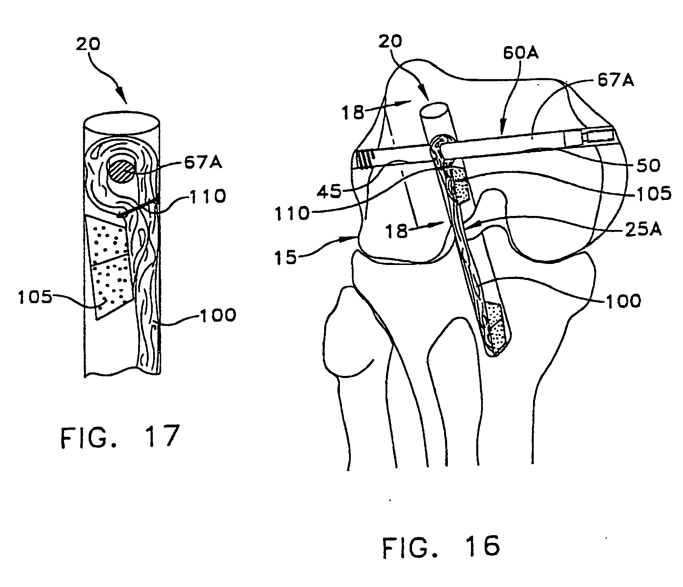

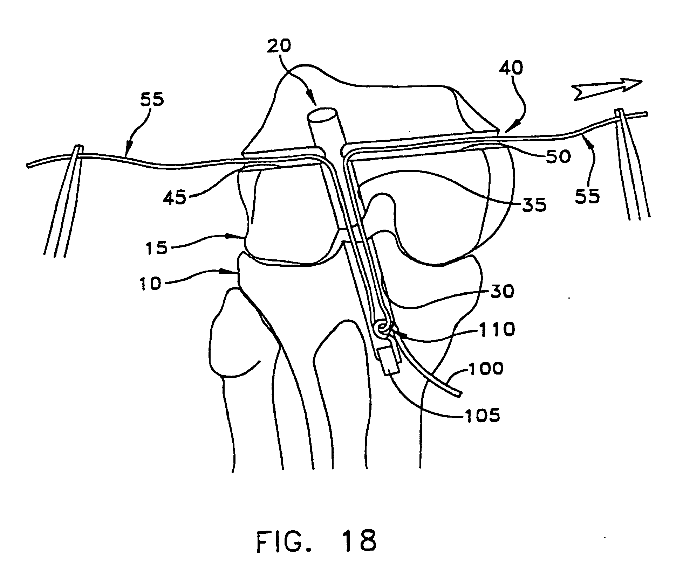

Furthermore, and looking now at Figs. 16 and 17, if desired, a suture 110 may be used to hold the graft ligament in a looped form. Again, Fig. 18 illustrates how graft ligament 25A may be loaded onto flexible member 55 so that it subsequently achieves the position shown in Figs. 16 and 17 with respect to crosspin 60A.

With respect to the bone-block-based crosspinning techniques described above and illustrated in Figs. 10-18, it should also be appreciated that while the techniques have been discussed in the context of the aforementioned crosspin 60A, they may also be practiced equally well with the crosspins 60 and 60B discussed above, as well as with other crosspins well known in the art.

### Crosspinning Procedure Utilizing A Plate

### And A Graft Ligament Comprising A Bone Block

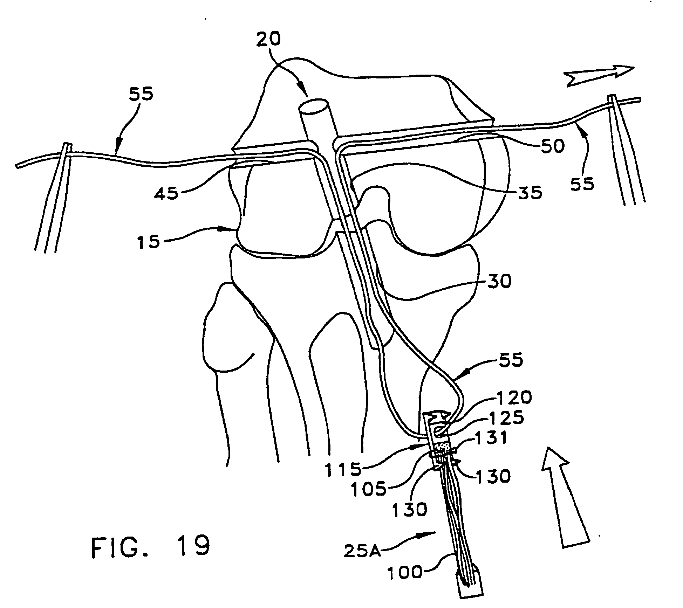

Looking now at Figs. 19-21, there is shown a plate 115 which may be used to secure bone block 105 of graft ligament 25A to femur 15. Plate 115 comprises a body portion 120, an opening 125 formed in body portion 120, and a plurality of pointed legs 130.

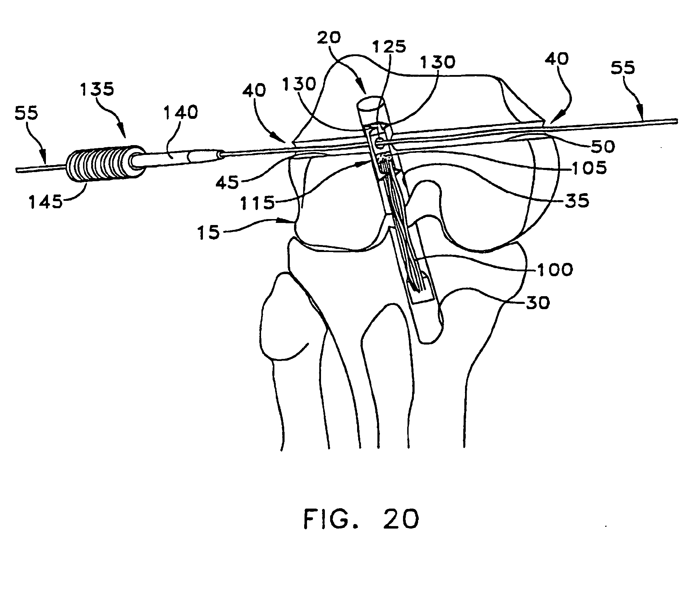

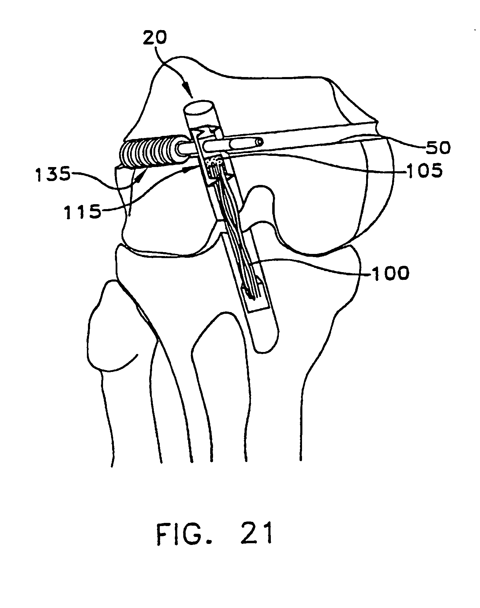

Plate 200 is arranged to have flexible member 55 passed through opening 125, and bone block 105 seated against body portion 120 (Fig. 19), with or without a securing suture 131, whereby flexible member 55 may be used to draw plate 115, and hence graft ligament 25A, up into position in femur 15 (Fig. 20). Thereafter, a cannulated crosspin 135, comprising a shaft 140 and enlarged screw threads 145, is passed over flexible member 55 and into first transverse bone tunnel portion 45. Crosspin 135 is then advanced within transverse bone tunnel 40 so that the crosspin's shaft 140 passes through opening 125 in plate 115 and into second transverse bone tunnel portion 50, and so that the crosspin's enlarged screw threads 145 engage the outside surface of femur 15. A cannulated driver (not shown) is then used to advance crosspin 135 further into transverse bone tunnel 40. By sizing the plate's opening 120 so that it will make a snug fit with the crosspin's shaft 140, the crosspin will drive plate 115 laterally, whereby to drive the plate's pointed legs' 130 into the side wall of femoral tunnel 35, and whereby to hold bone block 105 securely against the side wall of the bone tunnel (Fig. 21). Flexible member 55 may thereafter be removed so as to complete the crosspinning procedure.

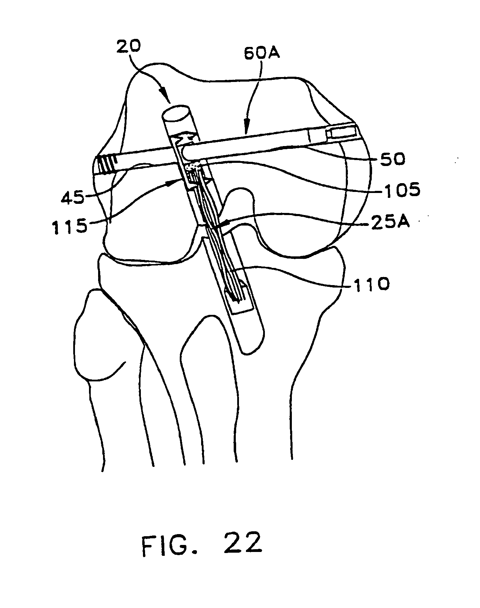

Looking next at Fig. 22, it is also possible to use crosspin 60A in conjunction with plate 115. In this case it is desirable to size the plate's opening 125 so that it will make a snug fit with the shaft of crosspin 60A, whereby the crosspin will drive the plate laterally against the side wall of femoral tunnel 35, whereby to fix graft ligament 25A in position.

With respect to the plate-and-bone-block-based crosspinning techniques described above and illustrated in Figs. 19-22, it should also be appreciated that while the techniques have been discussed in the context of the aforementioned crosspins 135 and 60A, they may also be practiced equally well with the crosspins 60 and 60B discussed above, as well as with other crosspins well known in the art.

The inventions discussed in the preceding sections can be comprised of any material applicable to orthopedic fixation devices such as implantable metallic, polymeric, composite, biologic or ceramic materials. However, in the case of the non-cannulated crosspins 60 and 60A, the solid cross-section unique to non-cannulated devices provides shear strength greater than that of similar diameter cannulated devices. Connection features, such as the openings 70 and threads 90, allow the non-cannulated crosspins 60 and 60A to be pulled through the knee and placed without any of the cross-sectional area being used for guidance tools such as the flexible member 55. This additional cross-sectional area helps to strengthen the portion of the crosspin that is under load by the ACL graft in actual clinical use. This consequently allows the non-cannulated crosspin with solid cross-sectional area to be made from a material which is typically weaker in shear strength than metal, such as non-absorbable or absorbable polymeric, composite, biologic or ceramic biomaterials, without significantly compromising the crosspin holding strength.

## Claims

1. A crosspin (60A) for supporting a graft ligament (25) in a first bone tunnel (20) formed in a bone (10, 15), by positioning the cross pin (60A) in a second bone tunnel (50) extending transverse to, and intersecting, the first bone tunnel (20), the crosspin comprising:
a shaft (65) having a distal portion (66A), an intermediate portion (67A), and a proximal portion (68);
the distal portion (66A) comprising attachment means (70) for attaching a flexible member (55) to said shaft (65);
the intermediate portion (67A) comprising screw threads (75); and
the proximal portion (68) including driver engagement means (80) for engagement by a driver (85) adapted to turn said shaft (65);
whereby the crosspin (60A) may be drawn through the second transverse bone tunnel (50) by the flexible member (55) attached to the distal portion (66A) by said attachment means (70), and
the crosspin (60A) being turnable to have screw threads (75) thereof turned into bone (15) by the driver (85) engaged with the engagement means (80);
**characterized by**
a a male/female screw mount (90) for releasably connecting the distal portion (66A) to the intermediate portion (67A), such that the distal portion (66A) is separable from the intermediate portion (67A).

2. The crosspin according to claim 1, wherein the intermediate portion (67A) comprises a solid shaft (65), and further wherein said attachment means (70) comprises at least one opening (70) formed in the distal portion (66A).

3. The crosspin in accordance with claim 1 wherein the flexible member (55) is attachable to the distal portion (66A).

4. A crosspin according to claim 3 wherein said connector portion (90) comprises a screw mount.

5. The crosspin in accordance with claim 3 wherein the crosspin (60) is positionable in the second transverse bone tunnel (50) by loading the crosspin (60) on, and moving the crosspin along, the flexible member (55).

## Patentansprüche

1. Ein Querstift (60A) zum Halten eines Band-Transplantats (25) in einem ersten Knochentunnel (20), der in einem Knochen (10,15) ausgebildet ist, durch Positionieren des Querstifts (60A) in einem zweiten Knochentunnel (50), der sich zum ersten Knochentunnel (20) quer erstreckt und diesen kreuzt, der Querstift umfasst:
einen Schaft (65) mit einem vorderen Bereich (66A), einen mittleren Bereich (67A) und einen hinteren Bereich (68);
der vordere Bereich (66A) umfasst Befestigungsmittel (70) zum Befestigen eines flexiblen Elements (55) an diesem Schaft (65);
der mittlere Bereich (67A) weist Schraubgewinde (75) auf; und der hintere Bereich (68) umfasst Dreher-Eingriffs-Einrichtungen (80) für das Eingreifen eines Drehers (85), der dazu eingerichtet ist, diesen Schaft (65) zu drehen;
wobei der Querstift (60A) durch den zweiten quer verlaufenden Knochentunnel (50) gezogen werden kann, durch das flexible Element (55), das am vorderen Bereich (66A) befestigt ist, durch die Befestigungsmittel (70), und
der Querstift (60A) ist drehbar, um die Schraubgewinde (75) daran in den Knochen (15) zu drehen mittels des Drehers (85), welcher in die Eingriffs-Einrichtungen (80) eingreift;
**gekennzeichnet durch**
eine männlich/ weibliche Schraubverbindung (90) zum lösbaren Verbinden des vorderen Bereichs (66A) mit dem mittleren Bereich (67A), so, dass der vordere Bereich (66A) von dem mittleren Bereich (67A) abtrennbar ist.

2. Der Querstift gemäß Anspruch 1, wobei der mittlere Bereich (67A) einen massiven Schaft (65) umfasst, und wobei ferner das Befestigungsmittel (70) zumindest eine Öffnung (70) aufweist, welche im vorderen Bereich (66A) ausgebildet ist.

3. Der Querstift entsprechend Anspruch 1, wobei das flexible Element (55) am vorderen Bereich (66A) befestigbar ist.

4. Ein Querstift gemäß Anspruch 3, wobei der Verbindungsbereich (90) eine Schraubverbindung aufweist.

5. Der Querstift entsprechend Anspruch 3, wobei der Querstift (60) im zweiten quer verlaufenden Knochentunnel (50) positionierbar ist durch Laden des Querstifts (60) auf das flexible Element (55), und Bewegen des Querstifts entlang des flexiblen Elements (55).

## Revendications

1. Contre-goupille (60A) pour supporter un ligament greffé (25) dans un premier tunnel osseux (20) formé dans un os (10, 15) en positionnant la contre-goupille (60A) dans un second tunnel osseux (50) s'étendant de manière transversale à, et coupant, le premier tunnel osseux (20), la contre-goupille comprenant :
une tige (65) ayant une partie distale (66A), une partie intermédiaire (67A), et une partie proximale (68) ;
la partie distale (66A) comprenant des moyens de fixation (70) pour fixer un élément flexible (55) à ladite tige (65) ;
la partie intermédiaire (67A) comprenant des filetages de vis (75); et
la partie proximale (68) comprenant des moyens de mise en prise d'un organe de commande (80) pour la mise en prise par un organe de commande (85) adapté afin de faire tourner ladite tige (65) ;
moyennant quoi la contre-goupille (60A) peut être tirée à travers le second tunnel osseux transversal (50) par l'élément flexible (55) fixé sur la partie distale (66A) par lesdits moyens de fixation (70), et
la contre-goupille (60A) pouvant tourner de manière à avoir ses filetages de vis (75) entraînés en rotation dans l'os (15) par l'organe de commande (85) mis en prise avec les moyens de mise en prise (80);
**caractérisée par**:
un support de vis mâle/femelle (90) pour raccorder de manière amovible la partie distale (66A) à la partie intermédiaire (67A), de sorte que la partie distale (66A) est séparable de la partie intermédiaire (67A).

2. Contre-goupille selon la revendication 1, dans laquelle la partie intermédiaire (67A) comprend une tige pleine (65), et en outre dans laquelle lesdits moyens de fixation (70) comprennent au moins une ouverture (70) formée dans la partie distale (66A).

3. Contre-goupille selon la revendication 1, dans laquelle l'élément flexible (55) peut être fixé sur la partie distale (66A).

4. Contre-goupille selon la revendication 3, dans laquelle ladite partie de connecteur (90) comprend un support de vis.

5. Contre-goupille selon la revendication 3, dans laquelle la contre-goupille (60) peut être positionnée dans le second tunnel osseux transversal (50) en chargeant la contre-goupille (60) sur, et en déplaçant la contre-goupille le long, de l'élément flexible (55).
